# EUROPEAN PATENT APPLICATION

(11) **EP 4 134 642 A1**
(43) Date of publication of application: **15.02.2023**
(21) Application number: 20930411.2
(22) Date of filing: 21.04.2020
(51) Int. Cl.: G01J 5/00, G01K 13/00, G01K 15/00

(54) **INFRARED THERMOMETER AND FOREHEAD TEMPERATURE CORRECTION AND MEASUREMENT METHOD**

(30) Priority: 11.04.2020 CN 202010282298
(71) Applicant: Shenzhen Youngme Information Communication System Co., Ltd., Shenzhen, Guangdong 518000 (CN)
(72) Inventor: YANG, Shengzhou, Shenzhen, Guangdong 518000 (CN)
(74) Representative: Cabinet Chaillot
(86) International application number: PCT/CN2020/085776
(87) International publication number: WO 2021/203466

(57) **Abstract**

The invention relates to an infrared thermometer and a forehead temperature correction and measurement method. The infrared thermometer comprises a temperature sensor, a corrector, a processor, and a display device. The temperature sensor is configured for measuring an actual temperature. In a correction mode, the corrector records actual temperature of an ear and a forehead of a user as an ear temperature and a forehead temperature respectively. In a forehead temperature measurement mode, the processor generates a body temperature according to a measured forehead temperature and the forehead temperature correction value, and the display device displays the body temperature. The infrared thermometer and the forehead temperature correction and measurement method can realize accurate measurement of the forehead temperature.

## Description

### FIELD

The present invention relates to the field of infrared temperature measurement, in particular to an infrared thermometer and a forehead temperature correction and measurement method.

### BACKGROUND

Infrared thermometers are mainstream thermometers in the present market, and the body temperature measured by most infrared thermometers is an estimated value based on a measured forehead temperature, that is, the measured forehead temperature is not the actual body temperature. Due to the difference between the measured forehead temperature and the actual body temperature, there will be an error when the body temperature of different humans is calculated based on the measured forehead temperature.

### SUMMARY

The invention provides an infrared thermometer and a forehead temperature correction and measurement method, which can realize accurate measurement of forehead temperatures.

An infrared thermometer comprises a temperature sensor, a corrector, a processor, and a display device. The temperature sensor is configured for measuring an actual temperature, the corrector is electrically connected to the temperature sensor, and the processor is electrically connected to the corrector and the temperature sensor. In a correction mode, the corrector records an ear temperature and a forehead temperature respectively, and obtains a forehead temperature correction value according to a difference between the ear temperature and the forehead temperature. In a forehead temperature measurement mode, the processor is capable of generating a body temperature according to a measured forehead temperature obtained by the temperature sensor and the forehead temperature correction value, and the display device displays the body temperature. According to this design, during the correction process, the actually measured ear temperature and the actually measured forehead temperature are correlated and regarded as equivalents through the cooperation of the temperature sensor, the corrector, the processor, and the display device, that is, the actually measured forehead temperature is equivalent to an actually measured ear temperature; the display device displays the measured ear temperature of users as the body temperature; and after correction, the users can obtain an accurate body temperature by directly measuring the forehead temperature.

In an embodiment, the infrared thermometer further comprises a control device which comprises a first switch. When the first switch is turned on, the infrared thermometer enters the correction mode, and the corrector records a measured value as the ear temperature. When the first switch is turned off, the temperature sensor records another measured value as the forehead temperature, the corrector obtains the forehead correction value according to the difference between the ear temperature and the forehead temperature, and the infrared thermometer exits from the correction mode. The infrared thermometer is controlled to enter the correction mode through the first switch, such that an instruction can be sent clearly; and operation is easy.

In an embodiment, the infrared thermometer comprises a body and an forehead temperature cap, the body comprises a temperature measurement probe, the temperature sensor is located at a tail end of the temperature measurement probe, the first switch is installed on the body, and a protrusion is disposed on the forehead temperature cap. When the forehead temperature cap is snapped on the body, the protrusion presses the first switch, and the first switch is in an off state; and when the forehead temperature cap is separated from the body, the protrusion is moved away from the first switch, and the first switch is in an on state. The first switch is controlled by snapping the forehead temperature cap onto the body or separating the forehead temperature cap from the body, such that users can clearly distinguish a forehead temperature mode from an ear temperature mode during the correction process. Since only the forehead temperature needs to be measured in a later normal use process, users can turn off the switch to enter the forehead temperature measurement mode without removing the forehead temperature cap, so using is convenient.

In an embodiment, the control device further comprises a second switch; when the second switch is turned on, the infrared thermometer enters the correction mode, the corrector records a measured value as the ear temperature, and records another measured value within a preset time as the forehead temperature, and the corrector obtains the forehead correction value according to the difference between the ear temperature and the forehead temperature. The infrared thermometer can also enter the correction mode by controlling the second switch, the ear temperature and the forehead temperature are measured successively within a preset time for correction, multiple correction methods are provided, users can select the suitable one as needed, and the service life of the infrared thermometer is prolonged.

In an embodiment, the preset time is not over 30 seconds. After the ear temperature is measured, the forehead temperature will be measured when the preset condition is met, and the ear temperature and the forehead temperature of the same user can be obtained within a certain time interval, such that the influence of the difference in body temperature of the same user at different times in the day is reduced, the influence of the changes to the body temperature caused by environmental factors or body factors on the measurement is also reduced, and thus, the correction is accurate and reliable.

A forehead temperature correction and measurement method using the infrared thermometer comprises: recording, by the corrector, a measured value as an ear temperature; recording, by the corrector, another measured value as a forehead temperature; obtaining, by the corrector, a forehead temperature correction value according to a difference between the ear temperature and the forehead temperature; and generating, by the processor, a body temperature according to the measured forehead temperature and the forehead temperature correction value.

According to the forehead temperature correction and measurement method of an infrared thermometer, the corrector obtains the forehead temperature correction value according to the difference between the ear temperature and the forehead temperature, and after being corrected, the measured temperature is closer to the true body temperature; and the processor generates the body temperature according to the measured forehead temperature and the forehead temperature correction value, such that users can obtain the accurate body temperature only by measuring the forehead temperature during normal use.

In an embodiment, the method further comprises: recording user identity information by the processor, and associating the forehead temperature correction value with the user identity information; and the step of recording user identity information by the processor further comprises: setting a startup default, or setting APP control, or setting a response key, or setting finger recognition, or setting iris recognition. By associating the forehead temperature correction value with the user identity information, users can correct their forehead temperature by means of their ear temperature; when the infrared thermometer is used by different users, identity information of multiple users can be recorded, such that the problem of large errors of measured forehead temperatures of different users is solved; in addition, through the step of setting a startup default, or setting APP control, or setting a response key, or setting finger recognition, or setting iris recognition, users can input information accurately and conveniently.

In an embodiment, the method further comprises: after recording, by the corrector, a measured value as an ear temperature, recording another measured value as a forehead temperature when a preset condition is met, wherein the preset condition comprises a preset time. In this step, the forehead temperature and the ear temperature are measured within a certain time, such that inaccurate measurement caused by a large time interval between forehead temperature measurement and ear temperature measurement is avoided; and the ear temperature mode and forehead temperature measurement mode are switched when the preset condition is met, such that a sensor can clearly determine whether the ear temperature or the forehead temperature is measured, and the correction process is accurate and reliable.

In an embodiment, the preset time is not over 30 seconds. After the ear temperature is measured, the forehead temperature will be measured when the preset condition is met, and the ear temperature and the forehead temperature of the same user can be obtained within a certain time interval, such that the influence of the difference in body temperature of the same user at different times in the day is reduced, the influence of the changes to the body temperature caused by environmental factors or body factors on the measurement is also reduced, and thus, the correction is accurate and reliable.

In an embodiment, the method further comprises: acquiring multiple sets of ear temperatures and forehead temperatures; obtaining multiple forehead temperature correction values by means of the multiple sets of ear temperatures and forehead temperatures; calculating an average value of the multiple forehead temperature correction values, and; updating the forehead correction value with the average value. By using the average value of multiple correction values, the correction process is more accurate.

According to the infrared thermometer and the forehead temperature correction and measurement method, users can correct their forehead temperature by means of their ear temperature, such that the problem of large errors of measured forehead temperatures of different users is solved; and the corrector calculates the forehead temperature correction value according to the measured forehead temperature and the measured ear temperature, such that the measured temperature is closer to the true body temperature.

### BRIEF DESCRIPTION OF THE DRAWINGS

FIG. 1 is a structural diagram of an infrared thermometer according to an embodiment of the invention.
FIG. 2 is a connection diagram of components of the infrared thermometer according to an embodiment of the invention.
FIG. 3 is a work flow diagram of a forehead temperature correction and measurement method according to an embodiment of the invention.

### DESCRIPTION OF THE EMBODIMENTS

The technical solutions of the embodiments of the invention will be clearly and completely described below in conjunction with the drawings of these embodiments. Obviously, the embodiments in the following description are merely illustrative ones, and are not all possible ones of the invention. All other embodiments obtained by those ordinarily skilled in the art based on the following ones without creative labor should also fall within the protection scope of the invention.

It should be noted that, when one element is considered as being "connected" to the other element, it may be directly connected to the other element or an intermediate element may exist between these two elements. When one element is considered as being "disposed" on the other element, it may be directly disposed on the other element or an intermediate element may exist between these two elements.

Unless otherwise defined, all technical and scientific terms used in this specification have the same meanings as those commonly understood by those skilled in the art. The terms used in this specification of the invention are merely for the purpose of describing specific embodiments, and are not intended to limit the invention. The term "and/or" used in this specification includes any and all combinations of one or more relevant items listed.

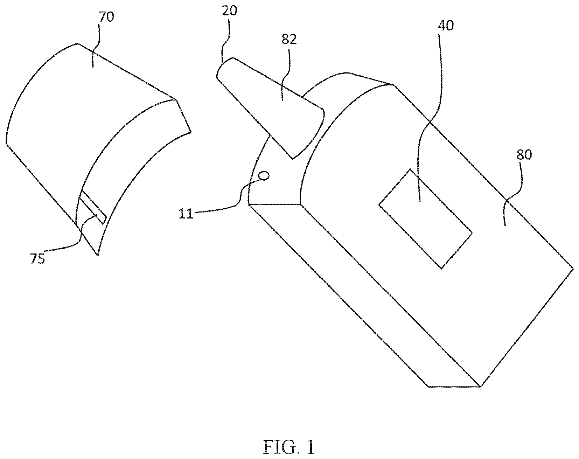

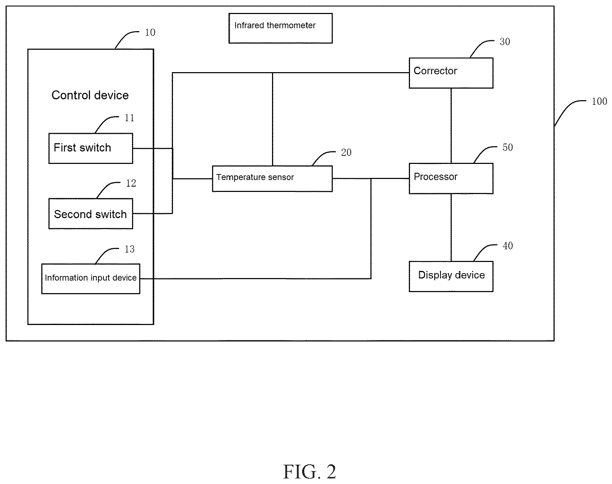

As shown in FIG. 1 and FIG. 2, an infrared thermometer 100 comprises a temperature sensor 20, a corrector 30, a processor 50, and a display device 40. The temperature sensor 20 is configured for measuring an actual temperature. The corrector 30 is electrically connected to the temperature sensor 20. The processor 50 is electrically connected to the corrector 30 and the temperature sensor 20. Before use, the infrared thermometer 100 is capable of performing a personal forehead temperature correction according to the features of the personal forehead temperature. In a correction mode, the corrector 30 records actual temperature of an ear and a forehead of a user as an ear temperature and a forehead temperature respectively. Specifically, the temperature sensor 20 successively performs temperature measurement twice, i.e., successively measuring an ear temperature and a forehead temperature for the user, and recognizes measurement results as the ear temperature and the forehead temperature respectively. The display device 40 displays the ear temperature and the forehead temperature in real time. The corrector 30 obtains a forehead temperature correction value according to a difference between the ear temperature and the forehead temperature. The display device 40 displays the forehead temperature correction value in real time. When the infrared thermometer 100 is used to measure a forehead temperature, the processor 50 generates a body temperature value according to the measured forehead temperature value measured by the temperature sensor 20 and the forehead temperature correction value. The display device 40 displays the body temperature value. The temperature sensor 20 may be an infrared sensor.

According to this design, during the correction process, the actually measured ear temperature and the actually measured forehead temperature are correlated and regarded as equivalents through the cooperation of the temperature sensor 20, the corrector 30, the processor 50, and the display device 40, that is, the actually measured forehead temperature is equivalent to an actually measured ear temperature; the display device 40 displays the measured ear temperature of users as the body temperature; and after correction, the users can obtain an accurate body temperature by directly measuring the forehead temperature.

In an embodiment, the infrared thermometer 100 further comprises a control device 10, wherein the control device 10 comprises a first switch 11; when the first switch 11 is turned on, the infrared thermometer 100 enters the correction mode, the corrector 30 records a measured value as the ear temperature; when the first switch 11 is turned off, the temperature sensor 20 records a measured value as the forehead temperature, the corrector 30 obtains the forehead temperature correction value according to the difference between the ear temperature and the forehead temperature, and then, the infrared thermometer 100 exits from the correction mode. The infrared thermometer 100 is controlled to enter the correction mode through the first switch 11, and operation is easy.

In an embodiment, the infrared thermometer 100 comprises a body 80 and a forehead temperature cap 70, wherein the body 80 comprises a temperature measurement probe 82, the temperature sensor 20 is located at a tail end of the temperature measurement probe 82, the first switch 11 is installed on the body 80, and a protrusion 75 is disposed on the forehead temperature cap 70. When the forehead temperature cap 70 is snapped on the body 80, the protrusion 75 presses the first switch 11, and the first switch 11 is in an off state. When the forehead temperature cap 70 is separated from the body 80, the protrusion 75 moves away from the first switch 11, and the first switch 11 is in an on state. The first switch 11 is controlled by snapping the forehead temperature cap 70 on the body 80 or separating the forehead temperature cap 70 from the body 80, such that users can clearly distinguish a forehead temperature mode from an ear temperature mode during the correction process. Since only the forehead temperature needs to be measured in a later normal use process, users can turn off the switch to enter the forehead temperature measurement mode without removing the forehead temperature cap 70, so using is convenient.

The temperature measurement probe 82 can be used for measuring both the ear temperature and the forehead temperature. When a preset condition is met, the temperature measurement probe 82 can measure the ear temperature and the forehead temperature successively. The preset condition comprises a preset time.

In an embodiment, the control device 10 further comprises a second switch 12. When the second switch 12 is turned on, the infrared thermometer 100 enters the correction mode, the temperature sensor 20 records a measured value as the ear temperature, and records again a measured value within a preset time as the forehead temperature, and the corrector 30 obtains the forehead temperature correction value according to the difference between the ear temperature and the forehead temperature. The infrared thermometer 100 can also enter the correction mode by controlling the second switch 12, the ear temperature and the forehead temperature are measured successively within a preset time for correction. Multiple correction methods are provided, users can select the suitable one as needed, and the service life of the infrared thermometer is prolonged.

The second switch 12 is a key-press response switch or a timing switch. The key-press response switch is controlled by users as needed. The timing switch does not need to be attended by users, and can remind users or be turned on or off automatically when the preset condition is met.

In an embodiment, the preset time is not over 60 seconds. Preferably, the preset time is not over 30 seconds. Specifically, the preset time may be 3 seconds, 5 seconds, 8 seconds, 15 seconds, 27 seconds, 30 seconds, 45 seconds, 50 seconds, or 60 seconds. After the ear temperature is measured, the forehead temperature will be measured when the preset condition is met, and the ear temperature and the forehead temperature of the same user can be obtained within a predetermined time interval, such that the influence of the difference in body temperature of the same user at different times in the day is reduced, the influence of the changes to the body temperature caused by environmental factors or body factors on the measurement is also reduced, and thus, the correction is accurate and reliable.

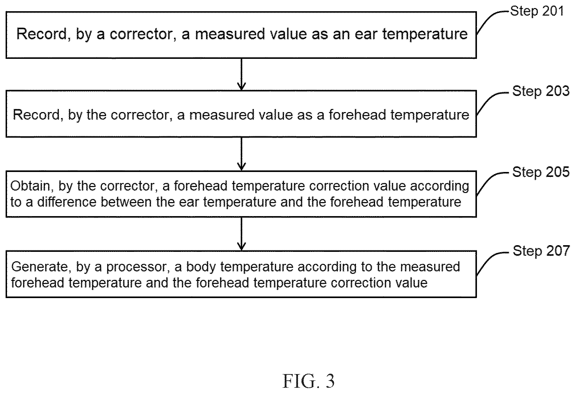

As shown in FIG. 3, a forehead temperature correction and measurement method comprises the following steps:
Step 201: recording, by a corrector, a measured value as an ear temperature;
Step 203: recording again, by the corrector, a measured value as a forehead temperature;
Step 205: obtaining, by the corrector, a forehead temperature correction value according to a difference between the ear temperature and the forehead temperature; and
Step 207: generating, by a processor, a body temperature according to the measured forehead temperature and the forehead temperature correction value.

According to the forehead temperature correction and measurement method of an infrared thermometer, the corrector obtains the forehead temperature correction value according to the difference between the ear temperature and the forehead temperature, and after being corrected, the measured temperature is closer to the true body temperature; and the processor generates the body temperature according to the measured forehead temperature and the forehead temperature correction value, such that users can obtain the accurate body temperature only by measuring the forehead temperature during normal use.

In an embodiment, the method further comprises: recording user identity information by the processor, and associating the forehead temperature correction value with the user identity information. The step of recording user identity information by the processor further comprises: setting a startup default, or setting APP control, or setting a response key, or setting finger recognition, or setting iris recognition. By associating the forehead temperature correction value with the user identify information, users can correct their forehead temperature by means of their ear temperature. When the infrared thermometer is used by different users, identity information of multiple users can be recorded, such that the problem of large errors of measured forehead temperatures of different users is solved. In addition, through the step of setting a startup default, or setting APP control, or setting a response key, or setting finger recognition, or setting iris recognition, users can input information accurately and conveniently.

In an embodiment, the method further comprises: after recording, by the corrector, a measured value as an ear temperature, recording again a measured value as a forehead temperature when a preset condition is met, wherein the preset condition comprises a preset time. In this step, the forehead temperature and the ear temperature are measured within a certain time, such that inaccurate measurement caused by a large time interval between forehead temperature measurement and ear temperature measurement is avoided; and the ear temperature mode and forehead temperature measurement mode are switched when the preset condition is met, such that a sensor can clearly determine whether the ear temperature or the forehead temperature is measured, and the correction process is accurate and reliable.

In an embodiment, the preset time is not over 30 seconds. In one embodiment, the preset time is not over 60 seconds.

In some embodiments, the preset time may be 3 seconds, 5, seconds, 8 seconds, 15 seconds, 27 seconds, 30 seconds, 45 seconds, 50 seconds, or 60 seconds. After the ear temperature is measured, the forehead temperature will be measured when the preset condition is met, and the ear temperature and the forehead temperature of the same user can be obtained within a certain time interval, such that the influence of the difference in body temperature of the same user at different times in the day is reduced, the influence of the changes to the body temperature caused by environmental factors or body factors on the measurement is also reduced, and thus, the correction is accurate and reliable.

In one embodiment, the method further comprises: acquiring multiple sets of ear temperatures and forehead temperatures, obtaining multiple forehead temperature correction values by means of the multiple sets of ear temperatures and forehead temperatures, calculating an average value of the multiple forehead temperature correction values, and updating the forehead correction value with the average value. By using the average value of multiple correction values, the correction process is more accurate.

According to the infrared thermometer and the forehead temperature correction and measurement method, users can correct their forehead temperature by means of their ear temperature, such that the problem of large errors of measured forehead temperatures of different users is solved; and after the ear temperature is measured, the forehead temperature will be measured when the preset condition is met, and the ear temperature and the forehead temperature of the same user can be obtained within a certain time interval, such that the influence of the difference in body temperature of the same user at different times in the day is reduced, and the influence of the changes to the body temperature caused by environmental factors or body factors on the measurement is also reduced. The corrector calculates the forehead temperature correction value according to the measured forehead temperature and the measured ear temperature within the certain time interval, such that the measured temperature is closer to the true body temperature. The process records the forehead temperature correction value and associates the forehead temperature correction value with user identity information to realize one-to-one correspondence of users and their body temperatures, such that the problem of errors of measured forehead temperatures of different users is solved. Further, accurate correction of the forehead temperature can be realized, and after correction, users can obtain an accurate body temperature by directly measuring the forehead temperature. Information of different users can be input to the infrared thermometer, different users correct their forehead temperature by means of their ear temperature, and can accurately measure their body temperature by selecting the corresponding user mode, and thus, the infrared thermometer can be used by multiple users.

The above embodiments merely illustrate several implementations of the invention and are specifically described in detail, but these embodiments should not be construed as limitations of the patent scope of the invention. It should be pointed out that some modifications and improvements can be made by those ordinarily skilled in the art without departing from the concept of the invention, and all these modifications and improvements should fall within the protection scope of the invention. Thus, the protection scope of the invention patent should be defined by the appended claims.

## Claims

1. An infrared thermometer, **characterized by** comprising: a temperature sensor, a corrector, a processor, and a display device,
wherein the temperature sensor is configured for measuring an actual temperature, the corrector is electrically connected to the temperature sensor, and the processor is electrically connected to the corrector and the temperature sensor;
in a correction mode, the corrector records an ear temperature and a forehead temperature respectively, and obtains a forehead temperature correction value according to a difference between the ear temperature and the forehead temperature; and
in a forehead temperature measurement mode, the processor is capable of generating a body temperature according to a measured forehead temperature obtained by the temperature sensor and the forehead temperature correction value, and the display device displays the body temperature.

2. The infrared thermometer according to Claim 1, **characterized in that** the infrared thermometer further comprises a control device which comprises a first switch;
when the first switch is turned on, the infrared thermometer enters the correction mode, and the corrector records a measured value as the ear temperature; and
when the first switch is turned off, the temperature sensor records another measured value as the forehead temperature, the corrector obtains the forehead correction value according to the difference between the ear temperature and the forehead temperature, and the infrared thermometer exits from the correction mode.

3. The infrared thermometer according to Claim 2, **characterized in that** the infrared thermometer comprises a body and an forehead temperature cap, the body comprises a temperature measurement probe, the temperature sensor is located at a tail end of the temperature measurement probe, the first switch is installed on the body, and a protrusion is disposed on the forehead temperature cap;
when the forehead temperature cap is snapped on the body, the protrusion presses the first switch, and the first switch is in an off state; and
when the forehead temperature cap is separated from the body, the protrusion is moved away from the first switch, and the first switch is in an on state.

4. The infrared thermometer according to Claim 2 or 3, wherein the control device further comprises a second switch; when the second switch is turned on, the infrared thermometer enters the correction mode, the corrector records a measured value as the ear temperature, and records another measured value within a preset time as the forehead temperature, and the corrector obtains the forehead correction value according to the difference between the ear temperature and the forehead temperature.

5. The infrared thermometer according to Claim 4, wherein the preset time is not over 30 seconds.

6. A forehead temperature correction and measurement method using the infrared thermometer according to Claim 1 or 2, **characterized by** comprising:
recording, by the corrector, a measured value as an ear temperature;
recording, by the corrector, another measured value as a forehead temperature;
obtaining, by the corrector, a forehead temperature correction value according to a difference between the ear temperature and the forehead temperature; and
generating, by the processor, a body temperature according to the measured forehead temperature and the forehead temperature correction value.

7. The forehead temperature correction and measurement method according to Claim 6, **characterized in that** the method further comprises: recording user identity information by the processor, and associating the forehead temperature correction value with the user identity information; and
wherein recording user identity information by the processor comprises: setting a startup default, or setting APP control, or setting a response key, or setting finger recognition, or setting iris recognition.

8. The forehead temperature correction and measurement method according to Claim 6, **characterized in that** the method further comprises: after recording, by the corrector, a measured value as an ear temperature, recording another measured value as a forehead temperature when a preset condition is met, wherein the preset condition comprises a preset time.

9. The forehead temperature correction and measurement method according to Claim 8, **characterized in that** the preset time is not over 30 seconds.

10. The forehead temperature correction and measurement method according to Claim 6, **characterized in that** the method further comprises:
acquiring multiple sets of ear temperatures and forehead temperatures;
obtaining multiple forehead temperature correction values by means of the multiple sets of ear temperatures and forehead temperatures;
calculating an average value of the multiple forehead temperature correction values, and;
updating the forehead correction value with the average value.
